# EUROPEAN PATENT APPLICATION

(11) **EP 1 832 817 A2**
(43) Date of publication of application: **12.09.2007**
(21) Application number: 07004612.3
(22) Date of filing: 06.03.2007
(51) Int. Cl.: F24F 3/16

(54) **Air filtering apparatus**

(30) Priority: 08.03.2006 JP 2006062173
(71) Applicant: SANYO ELECTRIC CO., LTD., Moriguchi-shi, Osaka (JP)
(72) Inventor: Nagae, Koji, Gunma 370-0514 (JP); Oya, Hiroshi, Ota-shi, Gunma 373-0842 (JP); Kondo, Yasuhito, Gunma 370-0514 (JP)
(74) Representative: Glawe, Delfs, Moll

(57) **Abstract**

An air filtering apparatus (1) including an electrolytic water mist generator (23) for generating electrolytic water mist from electrolytic water containing active oxygen specifies; and a blowing unit (7) for blowing air into the electrolytic watermist andblowingout air passed through the electrolytic water mist into a room.

## Description

### BACKGROUND OF THE INVENTION

### 1. Field of the Invention

The presents invention relates to an air filtering apparatus that can remove microorganisms such as bacteria, virus, fungus, etc. floating in the air (hereinafter merely referred to as "virus, etc.").

### 2. Description of the Related Art

There is generally proposed an air filtering apparatus for diffusing electrolytic water mist into the air, and bringing the electrolytic water mist into direct contact with virus, etc. to inactivate virus, etc. (JP-A-2002-181358).

The air filtering apparatus described above works effectively under a use atmosphere which fine particles of electrolytic water mist easily reach the whole area, that is, in a relatively small space, however, it hardly works effectively in a large space such as such as a kindergarten, an elementary/junior high/ high school, long-term care insurance facilities, a hospital or the like.

### SUMMARY OF THE INVENTION

Therefore, an object of the present invention is to provide an air filtering apparatus that can efficiently perform air filtering even in a large space under which electrolytic water mist hardly reaches the whole area.

In order to attain the above object, according to a first aspect of the present invention, an air filtering apparatus comprises: an electrolytic water mist generator for generating electrolytic water mist from electrolytic water containing active oxygen specifies; and an air blowing unit for blowing air into the electrolytic water mist and blowing out air passed through the electrolytic water mist into a room.

The above air-filtering apparatus may further comprise an air filtering chamber filled with the electrolytic water mist, wherein the blowing unit blows the air into the electrolytic water mist filled in the air filtering chamber.

Furthermore, the above air filtering apparatus may further comprise a heater for heating the air passed through the electrolytic water mist to vaporize water contained in the air passed through the electrolytic water mist.

The above air filtering apparatus may further comprise an air curtain generator for circulating the electrolytic water mist to generate electrolytic water mist air curtain, wherein the blowing unit blows the air so that the air passes through the electrolytic water mist air curtain.

The above air filtering apparatus may further comprise a rectifying member that suppresses disturbance of the flow of the electrolytic water mist air curtain due to the air blowing and is disposed along the flow of the electrolytic water mist air curtain.

The above air filtering apparatus may further comprise a filter disposed at an air blowing port from which the air-filtered air is blown to a room, the filter preventing passage of the electrolytic water mist and allowing passage of gas.

In the above air filtering apparatus, the electrolytic water mist generator may have an ultrasonic transducer for exciting the electrolytic water.

In the above air filtering apparatus, the electrolytic water mist generator may have an electrolytic unit for electrolyzing tap water to generate electrolytic water.

According to a second aspect of the present invention, an air filtering apparatus comprises a bubbling and blowing unit for bubbling electrolytic water containing active oxygen specifies by blowing air into the electrolytic water, and blowing the air passed through the electrolytic water to a room.

In the above air filtering apparatus, the bubbling and blowing unit may comprise a bubbling unit for bubbling electrolytic water containing oxygen specifies by blowing air into the electrolytic water, and an air blowing unit for blowing the air passed through the electrolytic water to the room.

The above air filtering apparatus may further comprise an electrolyzing unit for electrolyzing tap water to generate electrolytic water.

According to the present invention, when air is passed through electrolytic water mist or electrolytic water, virus, etc. contained in the air can be inactivated and air filtering can be efficiently performed even in a large space where electrolytic water mist hardly reaches virus, etc.

### BRIEF DESCRIPTION OF THE DRAWINGS

Fig. 1 is a perspective view showing an embodiment of the present invention;
Fig. 2 is a perspective view showing the internal construction;
Fig. 3 is a longitudinally sectional view of a housing;
Fig. 4 is a diagram showing the construction of a first embodiment;
Fig. 5 is a diagram showing the construction of a modification of the first embodiment;
Fig. 6 is a diagram showing the construction of a second embodiment;
Fig. 7 is a diagram showing the construction of a third embodiment;
Fig. 8 is a diagram showing the construction of a fourth embodiment; and
Fig. 9 is a diagram showing the construction of a fifth embodiment.

### DETAILED DESCRIPTION OF THE PREFERRED EMBODIMENTS

Preferred embodiments according to the present invention will be described hereunder with reference to the accompanying drawings.

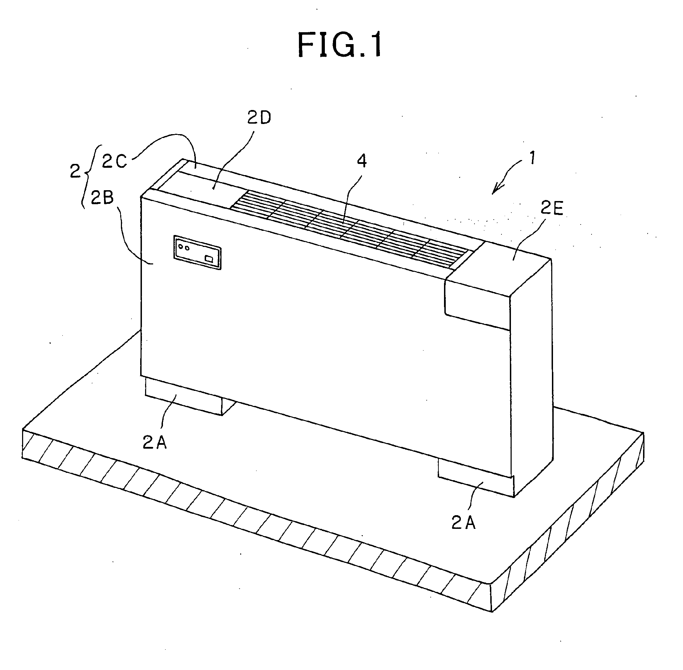

Fig. 1 is a perspective view showing an embodiment of the present invention

In Fig. 1, an on-floor mount type air filtering apparatus 1 has a box-shaped housing 2, and the housing 2 has leg pieces 2A, a front panel 2B and a top panel 2C. An operating lid 2D and an opening/closing lid 2E are juxtaposed with each other in the lateral direction at both the sides of the top panel 2C as shown in Fig. 1.

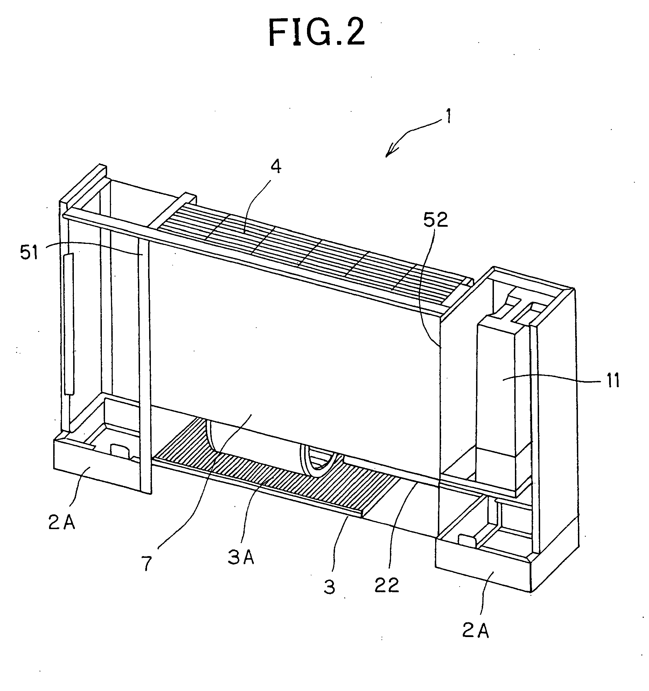

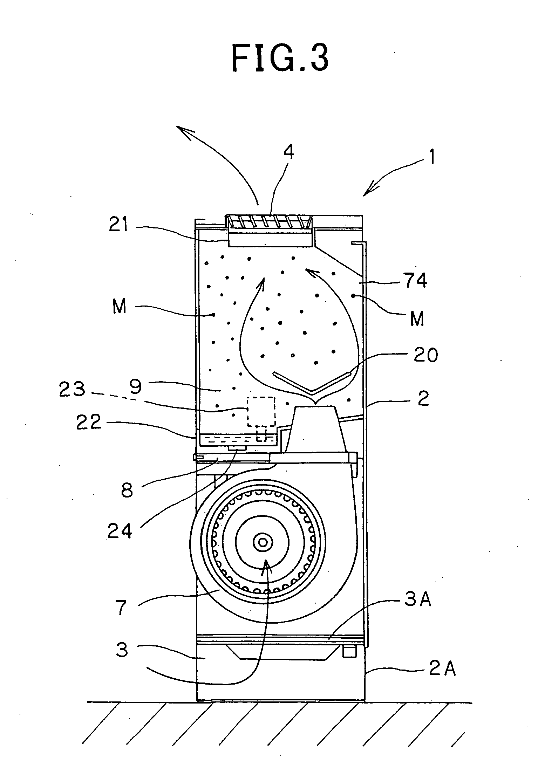

Fig. 2 is a perspective view showing the internal construction of the air filtering apparatus, and Fig. 3 is a longitudinally sectional view showing the housing.

As shown in Fig. 2, two partition plates 51 and 52 extending in the vertical direction are provided in the housing 2, and the inside of the housing 2 is divided into three chambers (an air filtering chamber 9, an electrical component chamber 61 and a water supply chamber 62) by the partition plates 51 and 52. A laterally elongated suction port 3 is formed at the lower portion of the air filtering chamber 9 formed at the center of the housing 2, and a pre-filter 3A is disposed above the suction port 3. An air blowing device 7 is supported above the pre-filter 3A through a support plate 8 by the partition plates 51, 52.

The air filtering chamber 9 is formed above the support plate 8. In the air filtering chamber 9 is provided a cover 20 which covers the upper side of the air blowing device 7 so that water droplets occurring from electrolytic water mist M are prevented from invading into an air blowing fan 7A constituting the air blowing device 7. A liquid passage preventing filter 21 for allowing only gas pass therethrough and preventing liquid containing the electrolytic water mist M from passing therethrough is disposed in the neighborhood of an air blowing port at the upper side of the air filtering chamber 9.

Furthermore, at the lower portion of the on-floor mount type air filtering apparatus 1 are provided a water stock tray 22 for stocking water such as tap water or the like supplied from a water supply tank 11 which stocks water W containing chlorine ions such as tap water or the like, an electrolytic unit 23 for electrolyzing water (tap water) W to generate electrolytic water EW, and an ultraviolet transducer 24 that is disposed at the lower surface of the water stock tray 22 and excites the electrolytic water EW in the water stock tray 22 to generate electrolytic mist M and fill the electrolytic mist M in the air filtering chamber 9. Furthermore, electrical parts (not shown) such as a print board for controlling the operation of the air filtering apparatus, etc. are accommodated in the electrical component chamber 61 formed at the left side of the housing 2.

### [1] First Embodiment

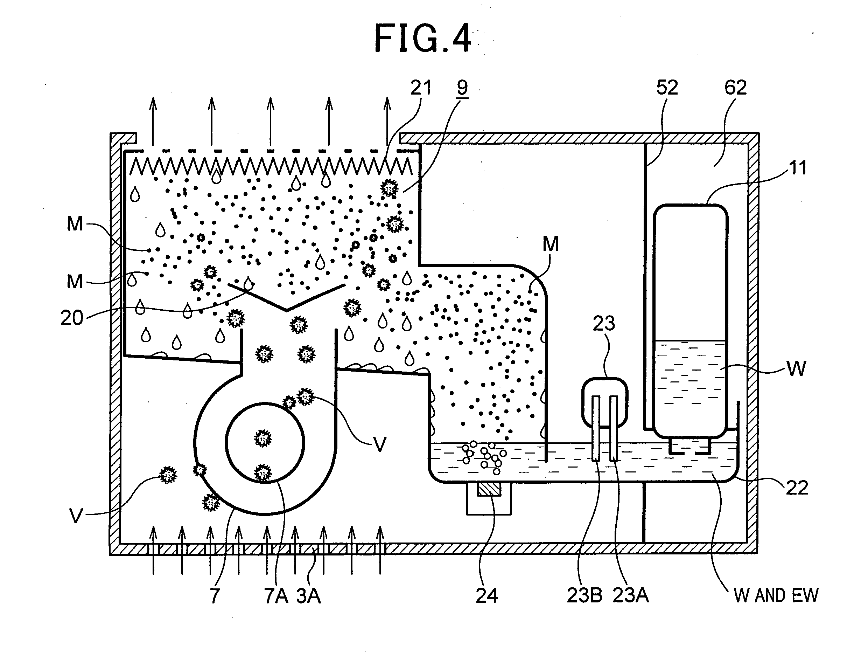

Fig. 4 is a diagram showing the construction of a first embodiment.

As shown in Fig. 4, an electrolytic bath unit 23 is disposed above the water stock tray 22, and equipped with plural pairs of electrodes 23A and 23B that serve as a cathode electrode and an anode electrode respectively and are alternately arranged (only two electrodes are illustrated in Fig. 4).

When current is supplied to the electrodes 23A and 23B, the water (tap water) W flowing into the water stock tray 22 is electrolyzed to generate active oxygen specifies. Here, the active oxygen specifies means oxygen molecules having higher oxidizing activity than normal oxygen and also related substance thereof, and contain not only so-called narrowly-defined active oxygen such as superoxide anion, singlet oxygen, hydroxyl radical and hydrogen peroxide, but also so-called broadly-defined active oxygen such as ozone, hypohalogen acid (hypochlorous acid), etc.

The electrodes 23A, 23B are electrode plates each of which comprises a base of Ti (titan) and a coated layer of Ir (iridium), Pt (platinum). The current value applied to the electrodes 23A, 23B is set so that a current density of several mA (milliampere)/cm² (square centimeter) to several tens mA/cm² is achieved and a predetermined concentration of free residual chlorine (for example, 1 mg (milligram)/1 liter) is generated, for example.

When current is supplied to tap water by the electrodes 23A, 23B, the following reaction occurs at the cathode:

4H⁺ + 4e⁻ + (4OH⁻) → 2H₂ + (4OH⁻)

Furthermore, the following reaction occurs at the anode:

2H₂O → 4H⁺ + O₂ + 4e⁻

At the same time, chlorine ions contained water (materials contained in tap water in advance) reacts as follows:

2Cl⁻ → Cl₂ + 2e⁻

Furthermore, Cl₂ thus generated reacts with water as follows:

Cl₂ + H₂O → HClO + HCl

Accordingly, by supplying current to the electrodes 23A, 23B, HClO (hypochlorous acid) having strong sterilizing force is generated. Therefore, indoor air blown from the fan device 7 is passed through the electrolytic water mist EW containing hypochlorousacid,therebyinactivating virus, etc. floating in the passing air. Furthermore, when odor components pass through the electrolytic water mist EW, the odor components also react with hypochlorous acid in the electrolytic water mist, and they are ionized and dissolved, whereby the air is deodorized.

Next, the air filtering operation of the on-floor mount type air filtering apparatus according to the first embodiment will be described.

In Fig. 1, an operation panel (not shown) which is exposed when the operating lid 2D is opened is provided inside the apparatus. By operating the operation panel, the driving of the on-floor mount type air filtering apparatus 1 is started. When this driving is started, the electrolytic unit 23 is driven as shown in Fig. 4, and the water (tap water) stocked in the water stock tray 22 is electrolyzed by supplying current to the electrodes 23A, 23B to generate electrolytic water EW containing active oxygen specifies. The electrolytic water EW is excited by the ultrasonic transducer 24, and filled as electrolytic water mist M in the air filtering chamber 9.

Under this state, air such as indoor air or the like is supplied through the air blowing fan 7 into the air filtering chamber 9. The indoor air is mixed with the electrolytic water mist M in the air filtering chamber 9, brought into contact with active oxygen specifies at the mixing stage and then blown out into the room again. For example, when influenza virus floats in the indoor air, the active oxygen species have the function of destructing or vanishing (removing) proteins (spikes) of the virus concerned which are required for infection. When the surface proteins are destructed, the influenza virus and the receptors required for infection of the virus concerned are not coupled with each other, and thus the infection can be prevented.

Thereafter, the electrolytic water mist M and the air which has been subjected to air infection and deodorization reach the liquid passage preventing filter 21. Here, the air-filtered and deodorized air is passed through the liquid passage preventing filter 21, however, the electrolytic water EW and the electrolytic water mist M which are liquid are prevented from passing through the liquid passage preventing filter 21 to the room side, and thus returned to the water stock tray 22 again.

In this construction, water is supplied in a circulation system, and when the amount of water is reduced by vaporization or the like, a proper amount of water (tap water) in the water supply tank 11 is supplied to the water stock tray 22. The water supply tank 11 is disposed so as to be freely taken out by opening the opening/closing lid 2E (see Fig. 1). Accordingly, water (tap water) can be supplemented by taking out the water supply tank 11.

According to the first embodiment, electrolytic water mist M is filled in the air filtering chamber 9, indoor air is mixed with the electrolytic water mist M so as to be brought into contact with the active oxygen specifies in the electrolytic water mist M. Therefore, virus, etc. in the indoor air can be efficiently inactivated (sterilized, removed, etc.), and thus the air filtering efficiency can be enhanced. Furthermore, deodorization can be also performed, and thus a comfortable indoor space can be constructed.

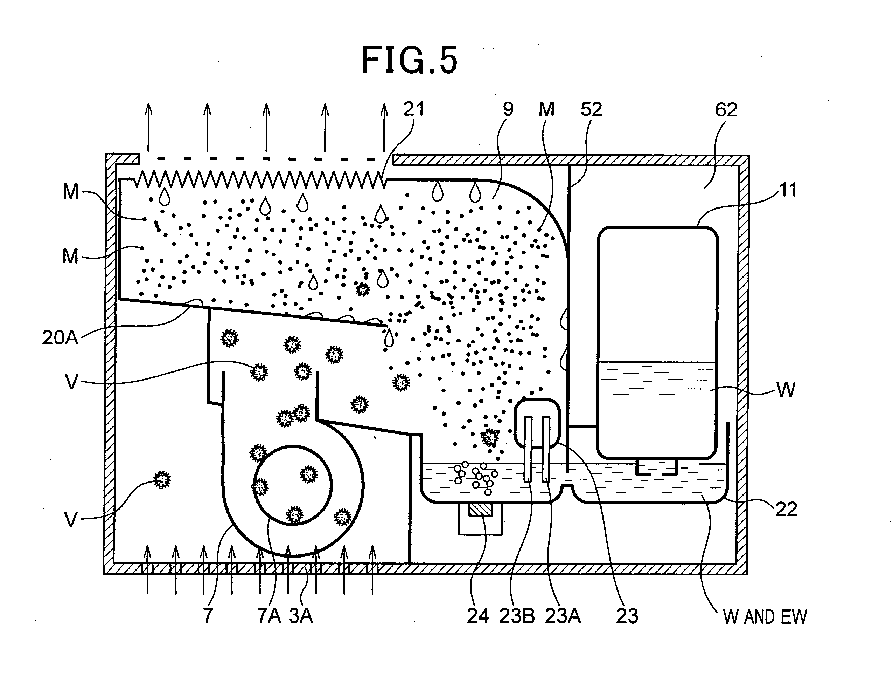

Fig. 5 is a diagram showing the construction of a modification of the first embodiment.

This modification is different from the first embodiment in that in place of the cover 20, the bottom plate of the air filtering chamber 9 is designed as a slant bottom plate 20A having a slope surface which obliquely downwardly extends to the water stock tray 22 side.

Accordingly, according to this modification, water droplets caused by the electrolytic water mist M after air filtering are prevented from invading into the air blowing fan 7A constituting the air blowing device 7, and also the water droplets are returned along the slant bottom plate 20A to the water stock tray 22 again, so that the use efficiency of the electrolytic water EW can be enhanced. The other effects are the same as the first embodiment.

### [2] Second Embodiment

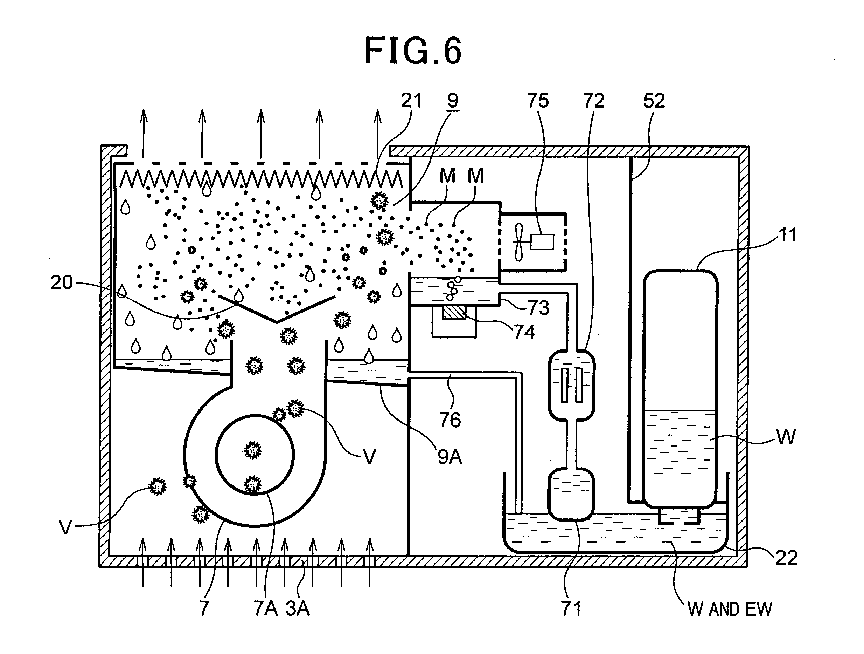

Fig. 6 is a diagram showing the construction of a second embodiment.

The second embodiment is different from the first embodiment in that water such as tap water is supplied to the electrolytic unit by a pump, and also a mist blowing fan for blowing electrolytic water mist M into the air filtering chamber is provided.

In the following description, the same parts as the first embodiment will be described with reference to the figures of the first embodiment.

In this embodiment, two partition plates 51 and 52 extending in the vertical direction are also provided inside the housing 2 as shown in Fig. 2, and the inside of the housing 2 is divided into three chambers (an air filtering chamber 9, an electrical component chamber 61 and a water supply chamber 62) by the partition plates 51 and 52. A laterally elongated suction port 3 is formed at the lower portion of the air filtering chamber 9 formed at the center of the housing 2, and a pre-filter 3A is disposed above the suction port 3. An air blowing device 7 is supported above the pre-filter 3A through a support plate 8 by the partition plates 51, 52.

The air filtering chamber 9 is formed above the support plate 8. In the air filtering chamber 9 is provided a cover 20 which covers the upper side of the air blowing device 7 so that water droplets occurring from electrolytic water mist M are prevented from invading into an air blowing fan 7A constituting the air blowing device 7. A liquid passage preventing filter 21 for allowing only gas pass therethrough and preventing liquid containing the electrolytic water mist M from passing therethrough is disposed in the neighborhood of an air blowing port at the upper side of the air filtering chamber 9.

At the lower portion of the on-floor mount type air filtering apparatus 1 are provided a water stock tray 22 for stocking water such as tap water or the like supplied from a water supply tank for stocking water (tap water) W containing chlorine ions, a pump 71 for pumping up the water W in the water stock tray 22, an electrolytic unit 72 for electrolyzing the water W pumped by the pump 71 to generate electrolytic water EW, an electrolytic water stocking portion 73 for stock the electrolytic water EW supplied by the electrolytic unit 72, an ultrasonic transducer 74 for exciting the electrolytic water EW in the electrolytic water stock portion 73 to generate electrolytic water mist M, a mist blowing fan 75 for blowing the generated electrolytic water mist M to fill the electrolytic water mist M in the air filtering chamber 9, and a return pipe 76 for returning to the water stock tray 22 electrolytic water EW caused by the electrolytic water mist M stocked at the bottom portion 9A of the air filtering chamber 9.

Next, the air filtering operation of the on-floor mount type air filtering apparatus of the second embodiment will be described.

In Fig. 1, by operating the operation panel which is provided inside the apparatus and exposed to the outside when the operating lid 2D is opened, the driving of the on-floor mount type air filtering apparatus 1 is started. When this driving is started, the pump 71 pumps up water (tap water) W in the water stock tray 22, and supplies the water to the electrolytic unit 72. The electrolytic unit 72 electrolyzes the water (tap water) W pumped and supplied by the pump 71 and generates electrolytic water W containing active oxygen species. The electrolytic water EW is excited by the ultrasonic transducer 24, and becomes electrolytic water mist M. In parallel to this process, the mist blowing fan 75 blows the generated electrolytic water mist M and fills the inside of the air filtering chamber 9 with the electrolytic water mist M.

Under this state, air such as indoor air or the like is supplied through the air blowing fan 7 into the air filtering chamber 9. The indoor air is mixed with the electrolytic water mist M in the air filtering chamber, brought into contact with active oxygen specifies at the mixing stage, and then blown out to the room again. For example, when influenza virus floats in the indoor air, the active oxygen species have the function of destructing or vanishing (removing) proteins (spikes) of the virus concerned which are required for infection. When the surface proteins are destructed, the influenza virus and the receptors required for infection of the virus concerned are not coupled with each other, and thus the infection can be prevented.

Thereafter, the electrolytic water mist M and the air which has been subjected to air infection and deodorization reach the liquid passage preventing filter 21. Here, the air-filtered and deodorized air is passed through the liquid passage preventing filter 21, however, the electrolytic water EW and the electrolytic water mist M which are liquid are prevented from passing through the liquid passage preventing filter 21 to the room side, stocked at the bottom portion 9A of the air filtering chamber 9, and then returned through the return pipe 76 to the water stock tray 22 again.

According to the second embodiment, the electrolytic water mist M is filled in the air filtering chamber 9 by the mist blowing fan 75, and the indoor air is stirred and mixed with the electrolytic water mist M so that the indoor air is surely brought into contact with the active oxygen specifies in the electrolyticwatermistM. Therefore, virus, etc. in the air (indoor air) can be efficiently inactivated and thus the air filtering efficiency can be enhanced. Furthermore, deodorization can be also performed, and thus a comfortable indoor space can be constructed.

### [3] Third Embodiment

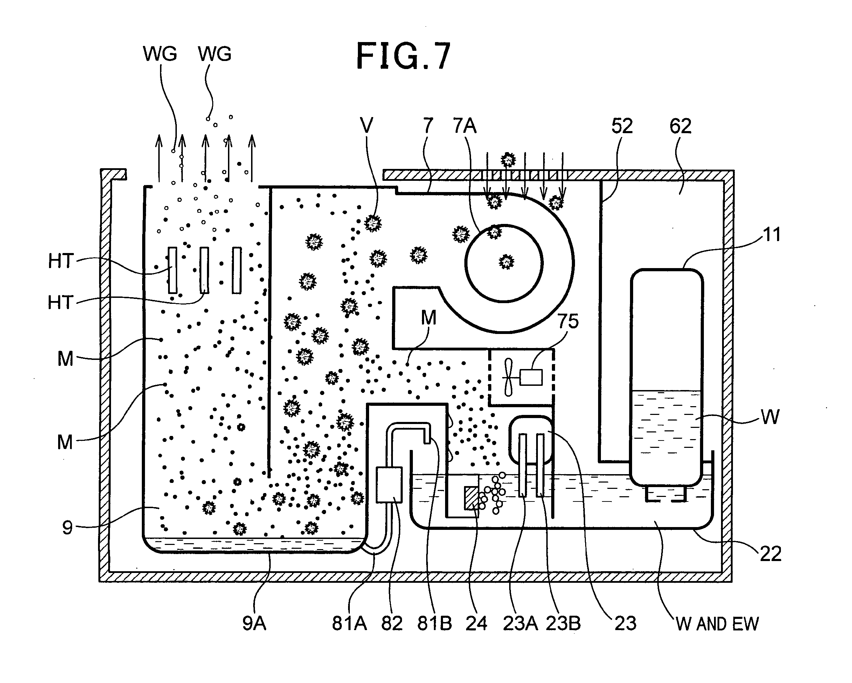

Fig. 7 is a diagram showing the construction of a third embodiment.

In the first and second embodiments, the electrolytic water mist M is prevented from being discharged to the room by the liquid passage preventing filter 21 after the air filtering is carried out by the electrolytic water mist M. However, the third embodiment is different from the first and second embodiments in that the electrolytic water mist after air filtering is heated and vaporized.

In the following description, the same parts as the first and second embodiments are represented by the same reference numerals and will be described with reference to the figures of the first and second embodiments.

Two partition plates 51 and 52 extending in the vertical direction are also provided inside the housing 2 as shown in Fig. 2, and the inside of the housing 2 is divided into three chambers (an air filtering chamber 9, an electrical component chamber 61 and a water supply chamber 62) by the partition plates 51 and 52. A laterally elongated suction port 3 is formed at the lower portion of the air filtering chamber 9 formed at the center of the housing 2, and a pre-filter 3A is disposed above the suction port 3. An air blowing device 7 is supported above the pre-filter 3A through a support plate 8 by the partition plates 51, 52.

At the lower portion of the on-floor mount type air filtering apparatus 1 are provided a water stock tray 22 for stocking water such as tap water or the like supplied from a water supply tank for stocking water (tap water) W containing chlorine ions, an electrolytic unit 23 for electrolyzing the tap water S in the water stock tray 22 to generate electrolytic water EW, an ultrasonic transducer 24 that is disposed at the lower surface of the water stock tray 22, excites the electrolytic water EW in the water stock tray 22 to generate electrolytic water mist M and fills the electrolytic water mist M in the air filtering chamber 9, a mist blowing fan 75 for blowing the generated electrolytic water mist M and filling the electrolytic water mist M in the air filtering chamber 9, a pump 82 for pumping electrolytic water EW caused by the electrolytic water mist M stocked at the bottomportion 9Aof the air filtering chamber 9 and returning the electrolytic water EW through return pipes 81A and 81B to the water stock tray 22, and a heater HT that is provided in the air filtering chamber 9 and heats the electrolytic water mist mixed with indoor air to generate steam WG.

Next, the operation of the on-floor mount type air filtering apparatus according to the third embodiment will be described.

In Fig. 1, by operating the operation panel which is provided inside the apparatus and exposed to the outside when the operating lid 2D is opened, the driving of the on-floor mount type air filtering apparatus 1 is started. When this driving is started, as shown in Fig. 4, the electrolytic unit 23 is driven, and current is supplied to the electrodes 23A and 23B, so that water (tap water) in the water stock tray 22 is electrolyzed and electrolytic water EW containing active oxygen specifies is generated. The electrolytic water EW is excited by the ultrasonic transducer 24, and becomes electrolytic water mist M. In parallel to this process, the mist blowing fan 75 blows the generated electrolytic water mist M so that the electrolytic water mist M is filled in the air filtering chamber 9.

Under this state, air such as indoor air or the like is supplied through the air blowing fan 7 into the air filtering chamber 9. The indoor air is mixed with the electrolytic water mist M in the air filtering chamber 9, and brought into contact with the active oxygen specifies at the mixing stage. For example, when influenza virus floats in the indoor air, the active oxygen species have the function of destructing or vanishing (removing) proteins (spikes) of the virus concerned which are required for infection. When the surface proteins are destructed, the influenza virus and the receptors required for infection of the virus concerned are not coupled with each other, and thus the infection can be prevented.

Thereafter, the air-filtered and deodorized air and the electrolytic water mist M reach the heater HT and are heated by the heater HT. The electrolytic water mist M is vaporized and discharged as steam WG.

At this time, the electrolytic water EW caused by the electrolytic water mist M stocked at the bottom portion 9A of the air filtering chamber 9 is pumped up by the pump 82, and returned through the return pipes to the water stock tray 22 again.

According to the third embodiment, the electrolytic water mist M is filled in the air filtering chamber 9 by the mist blowing fan 75, and the indoor air is stirred and mixed with the electrolytic water mist M so that the indoor air is surely brought into contact with the active oxygen specifies in the electrolytic water mist M. Therefore, virus, etc. in the indoor air can be efficiently inactivated, and thus the air filtering efficiency can be enhanced. Furthermore, deodorization can be also performed and a comfortable indoor space can be constructed.

At this time, the electrolytic water mist M after air filtering and deodorization is heated by the heater and supplied as steam to the room. Therefore, the humidity of the room can be properly kept, and breeding of virus, etc. can be suppressed. Therefore, the effect can be further enhanced together with the air filtering effect.

### [4] Fourth Embodiment

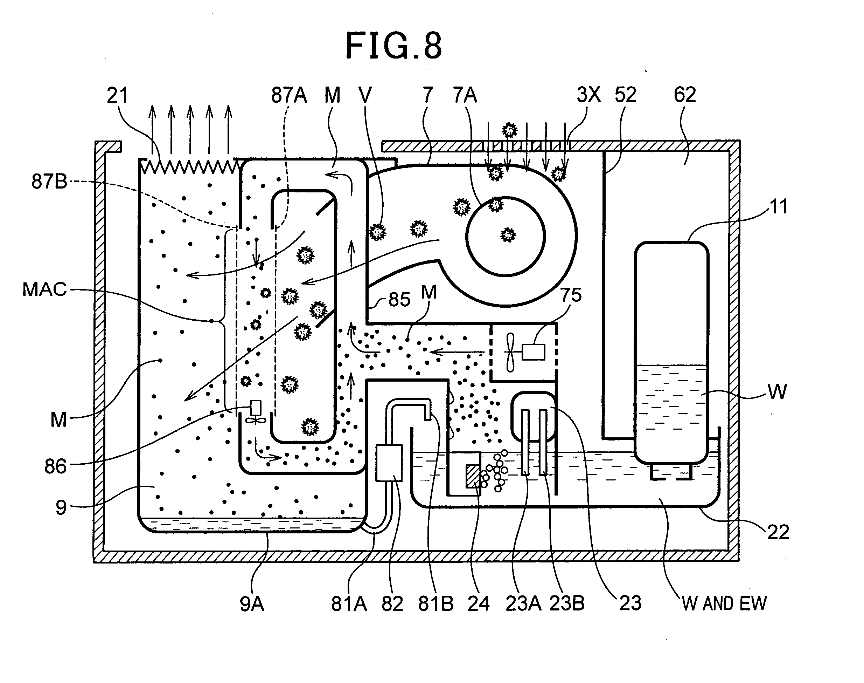

Fig. 8 is a diagram showing the construction of a fourth embodiment.

The fourth embodiment is different from the first to third embodiments in that the inside of the air filtering chamber 9 is not filled with electrolytic water mist M, but electrolytic water mist M is made to flow like a curtain to form an electrolytic mist air curtain MAC and air is passed through the electrolytic mist air curtain MAC to filter the air.

In the following description, the same parts as the first to third embodiments will be described with reference to the figures of the first to third embodiments.

Two partition plates 51 and 52 extending in the vertical direction are also provided inside the housing 2 as shown in Fig. 2, and the inside of the housing 2 is divided into three chambers (an air filtering chamber 9, an electrical component chamber 61 and a water supply chamber 62) by the partition plates 51 and 52. A laterally elongated suction port 3 is formed at the lower portion of the air filtering chamber 9 formed at the center of the housing 2, and a pre-filter 3A is disposed above the suction port 3. An air blowing device 7 is supported above the pre-filter 3A through a support plate 8 by the partition plates 51, 52.

A liquid passage preventing filter 21 for allowing passage of only gas and preventing passage of liquid containing electrolytic water mist M is disposed in the neighborhood of the air blowing port at the upper portion of the air filtering chamber 9.

Furthermore, at the lower portion of the on-floor mount type air filtering apparatus 1 are provided a water stock tray 22 for stocking water such as tap water or the like supplied from a water supply tank 11 which stocks water W such as tap water or the like containing chlorine ions, an electrolytic unit 23 for electrolyzing the water W in the water stock tray 22 to generate electrolytic water EW, an ultrasonic transducer 24 that is disposed at the lower surface of the water stock tray 22 and excites the electrolytic water EW in the water stock tray 22 to generate electrolytic water mist M and fill the electrolytic water mist M in the air filtering chamber 9, a mist blowing fan 75 for blowing the generated electrolytic water mist M to fill the electrolytic water mist in the air filtering chamber 9, and a pump 82 for pumping up electrolytic water EW caused by the electrolytic water mist M stocked at the bottom portion 9A of the air filtering chamber 9 and returns through the return pipes 81A, 81B to the water stock tray 22.

Furthermore, in the air filtering chamber 9 of the on-floor mount type air filtering apparatus 1 are provided a circulating duct 85 forming a flow path along which electrolytic water mist M is circulated to form the electrolytic mist air curtain MAC, a circulating blowing fan 86 for blowing air to circulate the electrolytic water mist M in the circulating duct 85 to form the electrolytic mist air curtain MAC, and two mesh-type rectifying members 87A and 87B that are disposed along the flow of the electrolytic mist air curtain and rectifies the flow of the electrolytic mist air curtain MAC so that the electrolytic mist air curtain MAC is not excessively disturbed by the blowing of air from the air blowing device 7.

Next, the air filtering operation of the on-floor mount type air filtering apparatus of the fourth embodiment will be described.

In Fig. 1, by operating the operation panel which is provided inside the apparatus and exposed to the outside when the operating lid 2D is opened, the driving of the on-floor mount type air filtering apparatus 1 is started. When this driving is started, the pump 71 pumps up the water (tap water) W in the water stock tray 22, and supplies the water W to the electrolytic unit 72.

The electrolytic unit 72 electrolyzes the supplied water (tap water) W and generates electrolytic water EW containing active oxygen specifies. The electrolytic water EW is excited by the ultrasonic transducer 24 and becomes electrolytic water mist M. In parallel to this process, the mist blowing fan 75 blows the generated electrolytic water mist M and supplies it to the circulating duct 85.

The electrolytic mist M supplied to the circulating duct 85 is circulated in the circulating duct 85 by the blowing of the circulating blowing fan 86, thereby forming the electrolytic mist air curtain MAC that is discharged from one end of the circulating duct 85 to the other end side thereof and passes through the gap between the rectifying members 87A and 87B.

Under this state, indoor air is passed through a suction port 3X at the top side and the air blowing fan 7, and supplied into the air filtering chamber 9. The indoor air successively passes through the rectifying member 87A, the electrolytic mist air curtain MAC and the rectifying member 87B in this order, whereby the indoor air is brought into contact with the active oxygen species in the electrolytic water mist in the electrolytic mist air curtain MAC as in the case of the first to third embodiments, thereby filtering and deodorizing the indoor air.

Thereafter, the electrolytic water mist M leaking from the electrolytic mist air curtain MAC through the rectifying member 87B and the air-filtered and deodorized air reaches the liquid passage preventing filter 21. At this time, the air-filtered and deodorized air passes through the liquid passage preventing filter. However, the electrolytic water EW and the electrolytic water mist M which are liquid are prevented from passing through the liquid passage preventing filter, and stocked at the bottom portion of the air filtering chamber 9.

At this time, the electrolytic water EW caused by the electrolytic water mist M stocked at the bottom portion 9Aof the air filtering chamber 9 is pumped up by the pump 82, and returned through the return pipes 81A and 81B to the water stock tray 22 again.

According to the fourth embodiment, indoor air is passed through the electrolytic water mist air curtain MAC, whereby the indoor air can be surely brought into contact with the active oxygen specifies in the electrolytic water mist M. Therefore, virus, etc. in the indoor air can be efficiently inactivated, and the air filtering efficiency can be enhanced. Furthermore, deodorization can be also performed. Accordingly, a comfortable indoor space can be constructed.

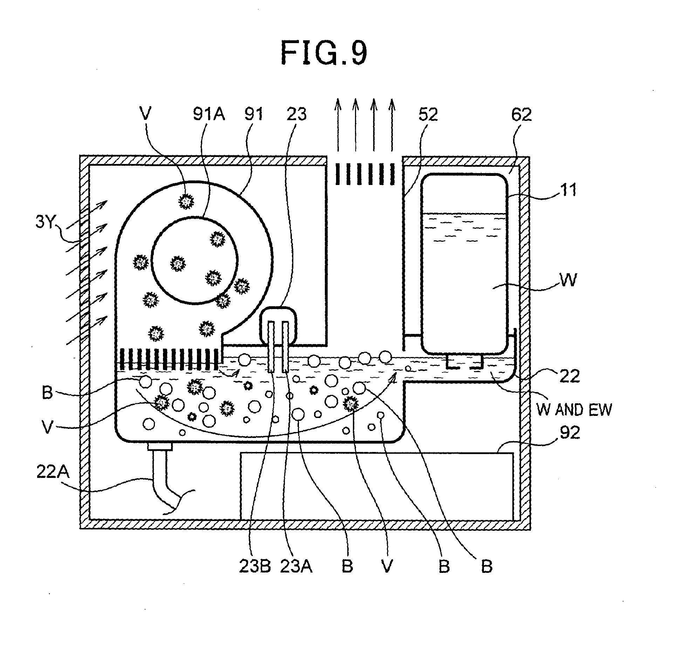

Fig. 9 is a diagram showing the construction of a fifth embodiment.

The fifth embodiment is different from the first to fourth embodiment in that air filtering is carried out not by using electrolytic water mist M, but by directly supplying air into the electrolytic water EW while making the air fine bubbles and passing air bubbles through the electrolytic water EW while stirring the electrolytic water EW, thereby performing air filtering.

In the following description, the same parts as the first to third embodiments will be described with reference to the figures of the first, second, third or fourth embodiment.

Two partition plates 51 and 52 extending in the vertical direction are also provided inside the housing 2 as shown in Fig. 2, and the inside of the housing 2 is divided into three chambers (an air filtering chamber 9, an electrical component chamber 61 and a water supply chamber 62) by the partition plates 51 and 52. A laterally elongated suction port 3 is formed at the lower portion of the air filtering chamber 9 formed at the center of the housing 2, and a pre-filter 3A is disposed above the suction port 3. A bubbling blowing device 91 having a bubbling blowing fan 91A is supported through the support plate 8 by the partition plates 51 and 52 and disposed above the pre-filter 3A.

At the lower portion of the on-floor mount type air filtering apparatus 1 are provided a water stock tray 22 that stocks water such as tap water supplied form a water supplying tank 11 for stocking water W such as tap water containing chlorine ions and is provided with a drain hose 22A at the lower surface thereof, an electrolytic unit 23 for electrolyzing the water W in the water stock tray 22 to generate electrolytic water EW, and a power source box 92 disposed below the water stock tray 22.

Furthermore, the air blowing port of the bubbling blowing device 91 is inserted in the electrolytic water EW stocked in the water stock tray 22 of the on-floor mount type air filtering apparatus 1. Accordingly, indoor air is blown through the air blowing port into the electrolytic water WE by the bubbling blowing device 91, and becomes bubbles B while stirred in the electrolytic water EW.

Next, the air filtering operation of the on-floor mount type air filtering apparatus according to the fifth embodiment will be described.

In Fig. 1, by operating the operation panel which is provided inside the apparatus and exposed to the outside when the operating lid 2D is opened, the driving of the on-floor mount type air filtering apparatus 1 is started. When this driving is started, the electrolytic unit 72 electrolyzes the water (tap water) W in the water stock tray 22 to generate electrolytic water EW containing active oxygen specifies.

Under this state, indoor air is passed through an air suction port 3Y at the front side and the bubbling blowing fan 91A of the bubbling blowing device 91 and supplied as fine bubbles into the electrolytic water EW in the water stock tray 22. The bubbles of indoor air are passed through the electrolytic water EW while stirred, and brought into contact with the active oxygen specifies in the electrolytic water EW as in the case of the first to fourth embodiments, thereby filtering and deodorizing the indoor air.

Thereafter, the filtered and deodorized air is fed out to the room.

According to the fifth embodiment, indoor air is passed as minute bubbles B through the electrolytic water EW while stirred, whereby the indoor air can be more surely brought into contact with the active oxygen specifies in the electrolytic water EW. Therefore, virus, etc. in the indoor air can be efficiently inactivated, and the air filtering efficiency can be enhanced. Furthermore, deodorization can be also performed. Therefore, a comfortable indoor space can be constructed.

Furthermore, in the fifth embodiment, the bubbling blowing fan 91A is used to supply air as bubbles into the electrolytic water EW. However, it may be modified so that indoor air is insufflated into electrolytic water EW and bubbled by using an air pump, and then air bubbles is supplied by the air blowing fan.

The present invention is not limited to the above-described embodiment. For example, the air filtering apparatus of the present invention may be designed so that ozone (O₃) or hydrogen peroxide (H₂O₂) is generated as the active oxygen species. In this case, when platinum tantalum electrodes are used as the electrodes, active oxygen species can be highly efficiently and stably generated from water in which ion species are rare.

At this time, at the anode, the following reaction occurs:

2H₂O → 4H⁺ + O₂ + 4e⁻

Simultaneously with the above reaction, the following reactions occur, and ozone (O₃) is generated.

3H₂O → O₃ + 6H⁺ + 6e⁻

2H₂O → O₃ + 4H⁺ + 4e⁻

Furthermore, at the cathode, the following reactions occur:

4H⁺ + 4e⁻ + (4OH⁻) → 2H₂ + (40H⁻)

O₂⁻ + e⁻ + 2H⁺ → H₂O₂

That is, O₂⁻ generated through the electrode reaction and H⁺ in solution are bonded to each other to generate hydrogen peroxide (H₂O₂).

In this construction, ozone (O₃) and hydrogen peroxide (H₂O₂) which have strong sterilizing power are generated by supplying current to the electrodes, and electrolytic water containing ozone (O₃) and hydrogen peroxide (H₂O₂) can be created. The concentration of ozone or hydrogen peroxide in the electrolytic water is adjusted to a value suitable for inactivate target virus or the like and air is passed through the gas-liquid contact member 5 supplied with the electrolytic water having this concentration, whereby target virus, etc. floating in the air can be inactivated. Furthermore, even odor reacts with ozone or hydrogen peroxide in the electrolytic water when passing through the gas-liquid contact member 5, and ionized and dissolved in the electrolytic water, whereby the odor components are removed from the air and thus the air is deodorized.

When scale is deposited on the electrode (cathode) due to electrolysis of tap water, it is difficult to continue the electrolysis because the electrical conductivity is lowered.

In this case, it is effective to invert the polarities of the electrodes (switching the plus and minus polarities of the electrodes). The scale deposited on the cathode can be removed by performing electrolysis while the cathode is used as the anode. Under the polarity inverting control, the polarities of the electrodes may be inverted periodically by using a timer, or the polarities of the electrodes may be inverted irregularly, for example, every time the operation of the apparatus is started or the like. Furthermore, increase of the electrolysis resistance (reduction in electrolysis current or increase in electrolysis voltage) may be detected, and the polarities of the electrodes may be inverted on the basis of the detection result.

The above embodiment adopts the water supply system based on the water supply tank 11 which can be freely put in and out. However, the present invention may adopt a water pipe water supply system in which a tap water pipe is connected to the apparatus in place of the water supply tank 11 and city water (tap water) is introduced.

## Claims

1. An air filtering apparatus comprising:
an electrolytic water mist generator for generating electrolytic water mist from electrolytic water containing active oxygen specifies; and
a blowing unit for blowing air into the electrolytic water mist and blowing out air passed through the electrolytic water mist into a room.

2. The air filtering apparatus according to claim 1, further comprising an air filtering chamber filled with the electrolytic water mist, wherein the blowing unit blows the air into the electrolytic water mist filled in the air filtering chamber.

3. The air filtering apparatus according to claim 1, further comprising a heater for heating the air passed through the electrolytic water mist to vaporize water contained in the air passed through the electrolytic water mist.

4. The air filtering apparatus according to claim 1, further comprising an air curtain generator for circulating the electrolytic water mist to generate electrolytic water mist air curtain, wherein the blowing unit blows the air so that the air passes through the electrolytic water mist air curtain.

5. The air filtering apparatus according to claim 4, further comprising a rectifying member that suppresses disturbance of the flow of the electrolytic water mist air curtain due to the air blowing and is disposed along the flow of the electrolytic water mist air curtain.

6. The air filtering apparatus according to claim 4, further comprising a filter disposed at an air blowing port from which the air-filtered air is blown to a room, the filter preventing passage of the electrolytic water mist and allowing passage of gas.

7. The air filtering apparatus according to claim 1, wherein the electrolytic water mist generator has an ultrasonic transducer for exciting the electrolytic water.

8. The air filtering apparatus according to claim 1, wherein the electrolytic water mist generator has an electrolytic unit for electrolyzing tap water to generate electrolytic water.

9. An air filtering apparatus comprising a bubbling and blowing unit for bubbling electrolytic water containing active oxygen specifies by blowing air into the electrolytic water, and blowing the air passed through the electrolytic water to a room.

10. The air filtering apparatus according to claim 9, wherein the bubbling and blowing unit comprises a bubbling unit for bubbling electrolytic water containing oxygen specifies by blowing air into the electrolytic water, and an air blowing unit for blowing the air passed through the electrolytic water to the room.

11. The air filtering apparatus according to claim 9, further comprising an electrolyzing unit for electrolyzing tap water to generate electrolytic water.
